(19) Europäisches Patentamt
European Patent Office
Office européen des brevets

(11) Publication number: **0 302 020 B1**

(12) **EUROPEAN PATENT SPECIFICATION**

(45) Date of publication of patent specification: **04.05.94**

(51) Int. Cl.$^5$: **C07D 211/58**

(21) Application number: **88810507.9**

(22) Date of filing: **25.07.88**

The file contains technical information submitted after the application was filed and not included in this specification

(54) **Process for the preparation of 2,2,6,6-tetramethyl-4-piperidyl-amines.**

(30) Priority: **30.07.87 IT 2151287**

(43) Date of publication of application:
**01.02.89 Bulletin 89/05**

(45) Publication of the grant of the patent:
**04.05.94 Bulletin 94/18**

(84) Designated Contracting States:
**BE DE FR GB IT NL**

(56) References cited:
**EP-A- 0 033 529**
**US-A- 3 480 635**
**US-A- 4 607 104**

(73) Proprietor: **CIBA-GEIGY AG**
**Klybeckstrasse 141**
**CH-4002 Basel(CH)**
(84) Designated Contracting States:
**BE DE FR GB NL**

(73) Proprietor: **CIBA-GEIGY S.p.A.**
**Strada Statale 233-Km. 20,5**
**Origgio(IT)**
(84) Designated Contracting States:
**IT**

(72) Inventor: **Cassandrini, Paolo, Dr.**
**Via Nino Scota, 11**
**I-40100 Bologna(IT)**
Inventor: **Righini, Giordano**
**Piazza della Repubblica 6**
**I-40050 Monte S. Pietro (BO)(IT)**
Inventor: **Vignali, Graziano**
**Via IV Novembre, 15**
**I-40037 Sasso Marconi (BO)(IT)**

**EP 0 302 020 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a novel and convenient process for the preparation of 2,2,6,6-tetramethyl-4-piperidylamines, which is based on the reductive amination of 2,2,6,6-tetramethyl-4-piperidone in the presence of platinum or palladium as hydrogenation catalyst, in the absence of a solvent or in the presence of a quantity of water less than 5 % by weight relative to the reaction mixture.

2,2,6,6-Tetramethyl-4-piperidylamines are compounds of considerable industrial interest, since they can be used for the preparation of light stabilizers for synthetic polymers.

Their preparation has therefore been extensively described in the patent literature; this involves the reductive amination of 2,2,6,6-tetramethyl-4-piperidone in an organic solvent or in water or in a water/alcohol mixture, in the presence of a hydrogenation catalyst such as platinum, palladium or nickel. In particular, US Patents 3,904,581, 4,104,248, 4,315,859, 4,326,063, 4,415,688 and 4,533,688, German Patent 3,007,996 and WO 81/1,706 describe the preparation of 2,2,6,6-tetramethyl-4-piperidylamines, using methanol as the reaction medium; in US Patents 4,605,743 and 4,607,104, the reaction is carried out in water or in water/alcohol mixtures.

The present invention relates to a process for the preparation of 2,2,6,6-tetramethyl-4-piperidylamines of the general formula (I)

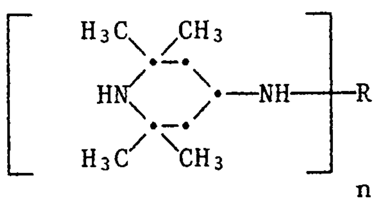

(I)

in which n is 1, 2 or 3 and, if n is 1, R is $C_1$-$C_{18}$-alkyl, $C_5$-$C_{12}$-cycloalkyl, $C_7$-$C_9$-phenylalkyl, tetrahydrofurfuryl, $C_2$-$C_4$-alkyl which is monosubstituted by OH, $C_1$-$C_8$-alkyloxy, $C_1$-$C_8$-alkylamino, cyclohexylamino or di($C_1$-$C_8$-alkyl)amino, or $C_2$-$C_6$-alkyl which is substituted by an $-NH_2$ group, or R is 2,2,6,6-tetramethyl-4-piperidyl, and, if n is 2, R is $C_2$-$C_{12}$-alkylene, cyclohexylene, methylenedicyclohexylene, cyclohexylenedimethylene, xylylene or $C_4$-$C_{12}$-alkylene which is interrupted by 1, 2 or 3 oxygen atoms or 1 or 2 >NH or >N-$CH_3$ groups, and, if n is 3, R is a group of the formula (II)

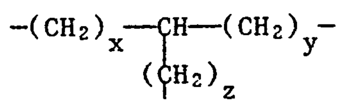

(II)

in which x and y are identical or different and are integers from 2 to 6 and z is zero or 1, characterized in that 2,2,6,6-tetramethyl-4-piperidone is reacted with an amine of the formula (III)

$R$-$(NH_2)_n$ (III)

in which R and n are as defined above, in the presence of hydrogen and of platinum or palladium as hydrogenation catalyst,
unsupported or supported on an inert material, in the absence of a solvent or in the presence of a quantity of water less than 5 % by weight relative to the reaction mixture.

The advantages of the procedure according to the present invention are clearly evident:

a) the absence of inflammable solvents, apart from representing an economic advantage, eliminates the fire risk connected with the use of pyrophoric catalysts, and

b) the absence of a solvent permits a significant increase in productive capacity per unit of reactor volume.

Representative examples of $C_1$-$C_{18}$-alkyl R are: methyl, ethyl, propyl, isopropyl, butyl, 2-butyl, isobutyl, t-butyl, pentyl, hexyl, octyl, 2-ethylhexyl, 1,1,3,3-tetramethylbutyl, nonyl, decyl, undecyl, dodecyl, tetradecyl, hexadecyl and octadecyl. $C_1$-$C_8$-alkyl is preferred.

Representative examples of $C_5$-$C_{12}$-cycloalkyl R are: cyclopentyl, cyclohexyl, methylcyclohexyl, dimethylcyclohexyl, trimethylcyclohexyl, cyclooctyl and cyclododecyl. A cycloalkyl group of the formula

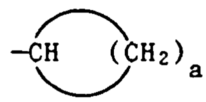

with a being an integer from 4 to 7 is preferred. Said group may optionally be substituted by $C_1$-$C_4$-alkyl, in particular methyl. Cyclohexyl is especially preferred.

Representative examples of $C_7$-$C_9$-phenylalkyl R are: benzyl, methylbenzyl, dimethylbenzyl and 2-phenylethyl. Benzyl unsubstituted or substituted at the phenyl ring by methyl is preferred.

Representative examples of $C_2$-$C_4$-alkyl R which is monosubstituted by OH are: 2-hydroxyethyl, 2-hydroxypropyl, 3-hydroxypropyl, 2-hydroxybutyl and 4-hydroxybutyl. 2-Hydroxyethyl is preferred.

Representative examples of $C_2$-$C_4$-alkyl R which is monosubstituted by $C_1$-$C_8$-alkyloxy are: 2-methoxyethyl, 2-ethoxyethyl, 2-butoxyethyl, 3-methoxypropyl, 3-ethoxypropyl, 3-propoxypropyl, 3-butoxypropyl, 3-hexyloxypropyl and 3-octyloxypropyl. 3-Methoxypropyl is preferred.

Representative examples of $C_2$-$C_4$-alkyl R which is monosubstituted by $C_1$-$C_8$-alkylamino or cyclohexylamino are: 2-methylaminoethyl, 3-methylaminopropyl, 3-ethylaminopropyl, 3-butylaminopropyl, 3-hexylaminopropyl, 3-octylaminopropyl and 3-cyclohexylaminopropyl.

Representative examples of $C_2$-$C_4$-alkyl R which is monosubstituted by di($C_1$-$C_8$-alkyl)amino are: 2-dimethylaminoethyl, 3-dimethylaminopropyl, 3-diethylaminopropyl, 3-dibutylaminopropyl, 3-dihexylaminopropyl and 3-dioctylaminopropyl. 3-Dimethylaminopropyl and 3-diethylaminopropyl are preferred.

Representative examples of $C_2$-$C_6$-alkyl R which is substituted by an $-NH_2$ group are: 2-aminoethyl, 2-aminopropyl, 3-aminopropyl, 4-aminobutyl, 5-aminopentyl, 6-aminohexyl and 3-amino-2,2-dimethylpropyl. 2-Aminoethyl is preferred.

Representative examples of $C_2$-$C_{12}$-alkylene R are: ethylene, propylene, trimethylene, tetramethylene, pentamethylene, 2,2-dimethyltrimethylene, hexamethylene, octamethylene, decamethylene and dodecamethylene. $C_2$-$C_6$-alkylene is preferred.

Representative examples of $C_4$-$C_{12}$-alkylene R which is interrupted by 1, 2 or 3 oxygen atoms are: 3-oxapentane-1,5-diyl, 4-oxaheptane-1,7-diyl, 3,6-dioxaoctane-1,8-diyl, 4,9-dioxadodecane-1,12-diyl and 4,7,10-trioxatridecane-1,13-diyl.

Representative examples of $C_4$-$C_{12}$-alkylene R which is interrupted by 1 or 2 $>NH$ or $>N-CH_3$ groups are: 3-azapentane-1,5-diyl, 3-azahexane-1,6-diyl, 4-azaheptane-1,7-diyl, 7-azatridecane-1,13-diyl, 4,7-diazadecane-1,10-diyl and 4-methyl-4-azaheptane-1,7-diyl.

Representative examples of a group R of the formula (II) are:

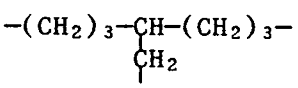 , 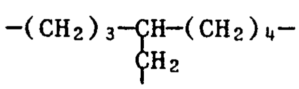 , 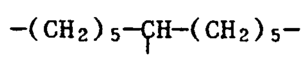 .

The process according to the present invention is conveniently carried out by reacting 2,2,6,6-tetramethyl-4-piperidone with an amine of the formula (III) in the presence of hydrogen and of platinum or palladium without an addition of solvents. The amine of the formula (III) is preferably an anhydrous amine, for example anhydrous butylamine.

In some cases a small quantity of water may be present, which originates e.g. from those amines of the formula (III) which are commercially available as concentrated aqueous solutions, in particular ethylamine containing 30 % of water and hexamethylenediamine containing 15 % of water. The water present in the mixture of the reagents is in any case less than 5 % relative to the weight of the said reagents.

The ratio of the 2,2,6,6-tetramethyl-4-piperidone and the amine of the formula (III) can be the theoretical ratio, or it is possible to use an excess of piperidone up to 20 % of theory, when n is 2 or 3, but, if n is 1, it is preferable to use an excess of amine up to 20 % of theory.

The catalysts which are used according to the process of the present invention are platinum or palladium, preferably platinum. The said catalysts can be used unsupported or supported on suitable inert materials, for example carbon, calcium carbonate, alumina and the like.

The reductive amination is conveniently carried out at a temperature of 20° to 120°C, preferably 40° to 100°C, under a hydrogen pressure of 1 to 100 bar, preferably 10 to 80 bar.

The process according to the present invention is possible because of the low melting point of 2,2,6,6-tetramethyl-4-piperidone (35°C) and the fact that the amines used as reagents for the reductive amination, and also the piperidylamines obtained, are usually liquid at room temperature or low-melting. For these

reasons, it is possible to react molten 2,2,6,6-tetramethyl-4-piperidone with the amine in liquid or solid form without any difficulties; once the reductive amination is complete, the catalyst can be separated off directly or after dilution with water.

Finally, the piperidylamines can be isolated by the usual procedures, for example by distillation or crystallization.

In general, the process according to the present invention can be carried out by introducing the molten 2,2,6,6-tetramethyl-4-piperidone into an autoclave in such a way that the initial temperature in the reactor is between 40° and 50°C. Then, the amine may be slowly added in liquid or solid form, or, in the case of methylamine, in gaseous form. The amine introduced is preferably at an initial temperature between 0° and 60°C, in particular between 20° and 40°C. During the addition of the amine, the reaction mixture is conveniently cooled, in order not to exceed 60°C.

After the addition of the amine, the catalyst can be introduced, preferably moistened with or suspended in water. The content of water added to the reaction mixture together with the catalyst is in general less than 2 % relative to the weight of the final reaction mixture. The reactor may be flushed several times with nitrogen and then pressurized with hydrogen and heated. When the absorption ceases, the reactor may be cooled to a temperature of 30-80°C, depending on the type of piperidylamine produced. The catalyst can be removed by filtration and the volatile products can be removed by distillation. Finally, the piperidylamine may be separated off by distillation or, when solid, crystallized, or used directly.

Those amines of the formula (III) are preferred, in which n is 1 or 2 and, if n is 1, R is $C_1$-$C_{12}$-alkyl, $C_5$-$C_9$-cycloalkyl, benzyl, tetrahydrofurfuryl, $C_2$-$C_3$-alkyl which is monosubstituted by OH, $C_1$-$C_4$-alkyloxy, $C_1$-$C_4$-alkylamino, cyclohexylamino or di($C_1$-$C_4$-alkyl)amino, or $C_2$-$C_6$-alkyl which is substituted by an -$NH_2$ group, or R is 2,2,6,6-tetramethyl-4-piperidyl, and, if n is 2, R is $C_2$-$C_6$-alkylene, cyclohexylene, methylenedicyclohexylene, cyclohexylenedimethylene or $C_4$-$C_{10}$-alkylene which is interrupted by 1 or 2 oxygen atoms or $>$NH groups.

Those amines of the formula (III) are particularly preferred, in which n is 1 or 2 and, if n is 1, R is $C_1$-$C_8$-alkyl, cyclohexyl, tetrahydrofurfuryl, 2,2,6,6-tetramethyl-4-piperidyl or $C_2$-$C_3$-alkyl which is monosubstituted by $C_1$-$C_4$-alkyloxy or by di($C_1$-$C_4$-alkyl)amino, and, if n is 2, R is $C_2$-$C_6$-alkylene or $C_4$-$C_{10}$-alkylene which is interrupted by 1 or 2 oxygen atoms or $>$NH groups.

Amines of the formula (III) of special interest are those, in which n is 1 or 2 and, if n is 1, R is $C_1$-$C_8$-alkyl, cyclohexyl, 2,2,6,6-tetramethyl-4-piperidyl, 3-methoxypropyl, 3-dimethylaminopropyl or 3-diethylaminopropyl, and, if n is 2, R is $C_2$-$C_6$-alkylene.

Amines of the formula (III) of particular interest are those, in which n is 1 or 2 and, if n is 1, R is $C_1$-$C_8$-alkyl or 3-diethylaminopropyl and, if n is 2, R is $-(CH_2)_{2-6}-$.

The amines of the formula (III), used according to the process of the present invention, are commercially available products or can be prepared by analogy to known processes. 2,2,6,6-Tetramethyl-4-piperidone can also be prepared by known processes, for example as described in US Patents 3,513,170, 3,959,295 and 4,536,581.

To illustrate the present invention more clearly, several examples of the preparation of 2,2,6,6-tetramethyl-4-piperidylamines are shown below.

Example 1: Preparation of 4-butylamino-2,2,6,6-tetramethylpiperidine

776.2 g (5 mol) of molten 2,2,6,6-tetramethyl-4-piperidone (temperature about 40°C) are introduced into a 2 litre autoclave and 402.3 g (5.5 mol) of butylamine are added with stirring, while maintaining the temperature at 30-40°C.

5 g of 5 % Pt on carbon, containing 50 % of water, are then added, and the hydrogenation is carried out at 70-80°C under a hydrogen pressure of 50 bar, until absorption ceases (4 hours). After cooling to ambient temperature, the catalyst is removed by filtration and the 4-butylamino-2,2,6,6-tetramethylpiperidine is separated off by distillation.

Boiling point: 154-156°C/66 mbar.
Yield: 98 % relative to 2,2,6,6-tetramethyl-4-piperidone.

Examples 2 to 8:

Following the procedure described in Example 1, the following piperidylamines of the formula

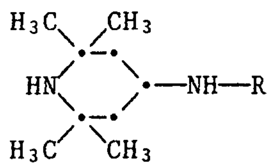

are prepared.

| Example | R | Boiling point (°C/mbar) | Yield (%) |
|---|---|---|---|
| 2 | $n-C_8H_{17}-$ | 143-145/4 | 95 |
| 3 | $CH_3O(CH_2)_3-$ | 90-93/0.5 | 92 |
| 4 | $C_4H_9CHCH_2-$<br>$C_2H_5$ | 147-149/5 | 93 |
| 5 | 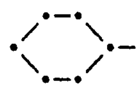 | 145-146/20 | 88 |
| 6 | $(C_2H_5)_2N-(CH_2)_3-$ | 143-145/0.8 | 96 |
| 7 | $(CH_3)_2N-(CH_2)_3-$ | 103-106/0.05 | 92 |
| 8 | $NH_2-(CH_2)_2-$ | 133-135/40 | 93 |

Example 9: Preparation of N,N'-bis-(2,2,6,6-tetramethyl-4-piperidyl)-trimethylenediamine

1,017.7 g (6.3 mol) of molten 2,2,6,6-tetramethyl-4-piperidone (temperature about 40 °C) are introduced into a 2 litre autoclave and 222.4 g (3 mol) of trimethylenediamine are added with stirring, while maintaining the temperature at about 40 °C.

6 g of 5 % Pt on carbon, containing 50 % of water, are then added, and the hydrogenation is carried out at 80 °C under a hydrogen pressure of 50 bar until absorption ceases (4-5 hours). The mixture is cooled to 60-70 °C, the catalyst is separated off by filtration and the N,N'-bis-(2,2,6,6-tetramethyl-4-piperidyl)-trimethylenediamine is isolated by distillation.
Boiling point: 171-173 °C/0.7 mbar.
Yield: 92 % relative to the 2,2,6,6-tetramethyl-4-piperidone.

Example 10: Preparation of N,N'-bis-(2,2,6,6-tetramethyl-4-piperidyl)-ethylenediamine

The product is obtained as described in the preceding example by hydrogenation at 50-60 °C under a hydrogen pressure of 50 bar.
Boiling point: 167-169 °C/0.7 mbar.
Yield: 90 % relative to the 2,2,6,6-tetramethyl-4-piperidone.

## Claims

**1.** A process for the preparation of 2,2,6,6-tetramethyl-4-piperidylamine of the formula (I)

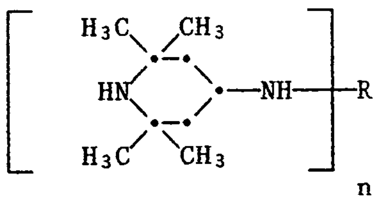

(I)

in which n is 1, 2 or 3 and, if n is 1, R is $C_1$-$C_{18}$-alkyl, $C_5$-$C_{12}$-cyclo-alkyl, $C_7$-$C_9$-phenylalkyl, tetrahydrofurfuryl, $C_2$-$C_4$-alkyl which is monosubstituted by OH, $C_1$-$C_8$-alkyloxy, $C_1$-$C_8$-alkylamino, cyclohexylamino or di($C_1$-$C_8$-alkyl)amino, or $C_2$-$C_6$-alkyl which is substituted by an -$NH_2$ group, or R is 2,2,6,6-tetramethyl-4-piperidyl, and, if n is 2, R is $C_2$-$C_{12}$-alkylene, cyclohexylene, methylenedicyclohexylene, cyclohexylenedimethylene, xylylene or $C_4$-$C_{12}$-alkylene which is interrupted by 1, 2 or 3 oxygen atoms or 1 or 2 $>$NH or $>$N-$CH_3$ groups, and, if n is 3, R is a group of the formula (II)

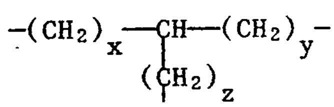

(II)

in which x and y are identical or different and are integers from 2 to 6 and z is zero or 1, characterized in that 2,2,6,6-tetramethyl-4-piperidone is reacted with an amine of the formula (III)

R-$(NH_2)_n$ (III)

in which R and n are as defined above, in the presence of hydrogen and of platinum or palladium as hydrogenation catalyst,
unsupported or supported on an inert material, in the absence of a solvent or in the presence of a quantity of water less than 5 % by weight relative to the reaction mixture.

**2.** A process according to claim 1, wherein the reductive amination of 2,2,6,6-tetramethyl-4-piperidone is carried out in the absence of a solvent.

**3.** A process according to claim 1, wherein the reductive amination of 2,2,6,6-tetramethyl-4-piperidone is carried out with an anhydrous amine of the formula (III).

**4.** A process according to claim 1, wherein the reductive amination of 2,2,6,6-tetramethyl-4-piperidone is carried out in the presence of a quantity of water less than 5 % by weight relative to the reaction mixture.

**5.** A process according to claim 1, wherein the reductive amination of 2,2,6,6-tetramethyl-4-piperidone is carried out with ethylamine containing 30 % of water.

**6.** A process according to claim 1, wherein the reductive amination of 2,2,6,6-tetramethyl-4-piperidone is carried out with hexamethylenediamine containing 15 % of water.

**7.** A process according to claim 1, wherein the reductive amination of 2,2,6,6-tetramethyl-4-piperidone is carried out in the presence of platinum as hydrogenation catalyst.

**8.** A process according to claim 1, wherein the reductive amination of 2,2,6,6-tetramethyl-4-piperidone is carried out at a temperature of 20° to 120°C.

9. A process according to claim 1, wherein the reductive amination of 2,2,6,6-tetramethyl-4-piperidone is carried out at a temperature of 40° to 100°C.

10. A process according to claim 1, wherein the reductive amination of 2,2,6,6-tetramethyl-4-piperidone is carried out under a hydrogen pressure of 1 to 100 bar.

11. A process according to claim 1, wherein the reductive amination of 2,2,6,6-tetramethyl-4-piperidone is carried out under a hydrogen pressure of 10 to 80 bar.

12. A process according to claim 1, wherein the reductive amination of 2,2,6,6-tetramethyl-4-piperidone is carried out by using an amine of the formula (III) in which n is 1 or 2 and, if n is 1, R is $C_1$-$C_{12}$-alkyl, $C_5$-$C_9$-cycloalkyl, benzyl, tetrahydrofurfuryl, $C_2$-$C_3$-alkyl which is monosubstituted by OH, $C_1$-$C_4$-alkyloxy, $C_1$-$C_4$-alkylamino, cyclohexylamino or di($C_1$-$C_4$-alkyl)amino, or $C_2$-$C_6$-alkyl which is substituted by an -$NH_2$ group, or R is 2,2,6,6-tetramethyl-4-piperidyl, and, if n is 2, R is $C_2$-$C_6$-alkylene, cyclohexylene, methylenedicyclohexylene, cyclohexylenedimethylene or $C_4$-$C_{10}$-alkylene which is interrupted by 1 or 2 oxygen atoms or >NH groups.

13. A process according to claim 1, wherein the reductive amination of 2,2,6,6-tetramethyl-4-piperidone is carried out by using an amine of the formula (III) in which n is 1 or 2 and, if n is 1, R is $C_1$-$C_8$-alkyl, cyclohexyl, tetrahydrofurfuryl, 2,2,6,6-tetramethyl-4-piperidyl or $C_2$-$C_3$-alkyl which is monosubstituted by $C_1$-$C_4$-alkyloxy or by di($C_1$-$C_4$-alkyl)amino, and, if n is 2, R is $C_2$-$C_6$-alkylene or $C_4$-$C_{10}$-alkylene which is interrupted by 1 or 2 oxygen atoms or >NH groups.

14. A process according to claim 1, wherein the reductive amination of 2,2,6,6-tetramethyl-4-piperidone is carried out by using an amine of the formula (III) in which n is 1 or 2 and, if n is 1, R is $C_1$-$C_8$-alkyl, cyclohexyl, 2,2,6,6-tetramethyl-4-piperidyl, 3-methoxypropyl, 3-dimethyl-aminopropyl or 3-diethylaminopropyl, and, if n is 2, R is $C_2$-$C_6$-alkylene.

15. A process according to claim 1, wherein the reductive amination of 2,2,6,6-tetramethyl-4-piperidone is carried out by using an amine of the formula (III) in which n is 1 or 2 and, if n is 1, R is $C_1$-$C_8$-alkyl or 3-diethylaminopropyl and, if n is 2, R is $-(CH_2)_{2-6}-$.

**Patentansprüche**

1. Verfahren zur Herstellung von 2,2,6,6-Tetramethyl-4-piperidylamin der Formel (I)

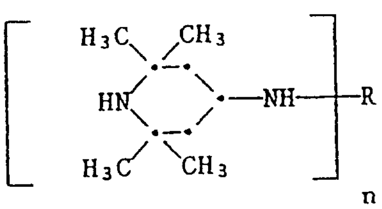

(I) .

worin n = 1, 2 oder 3, und falls n = 1 ist, ist R $C_1$-$C_{18}$--Alkyl, $C_5$-$C_{12}$-Cycloalkyl, $C_7$-$C_9$-Phenylalkyl, Tetrahydrofurfuryl, $C_2$-$C_4$-Alkyl, monosubstituiert durch OH, $C_1$-$C_8$-Alkyloxy, $C_1$-$C_8$-Alkylamino, Cyclohexylamino oder Di-($C_1$-$C_8$-alkyl)-amino, oder $C_2$-$C_6$-Alkyl, das durch eine -$NH_2$-Gruppe substituiert ist, oder R ist 2,2,6,6-Tetramethyl-4-piperidyl, und falls n = 2 ist, ist R $C_2$-$C_{12}$-Alkylen, Cyclohexylen, Methylendicyclohexylen, Cyclohexylendimethylen, Xylylen oder $C_4$-$C_{12}$-Alkylen, das durch 1, 2 oder 3 Sauerstoffatome oder 1 oder 2 >NH- oder >N-$CH_3$-Gruppen unterbrochen ist, und falls n = 3 ist, ist R eine Gruppe der Formel (II)

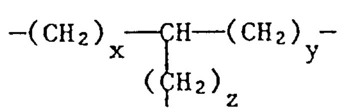

(II)

worin x und y identisch oder verschieden sind und Zahlen von 2 bis 6 sind und z ist 0 oder 1, dadurch

gekennzeichnet, daß 2,2,6,6-Tetramethyl-4-piperidon mit einem Amin der Formel (III)

$R-(NH_2)_n$ (III)

worin R und n wie vorstehend definiert sind, in Gegenwart von Wasserstoff und von Platin oder Palladium als Hydrierungskatalysator, selbsttragend bzw. ohne Träger oder auf einem - inerten Trägermaterial in Abwesenheit eines Lösungsmittels oder in Anwesenheit einer Wassermenge weniger als 5 Gew.-%, bezogen auf das Reaktionsgemisch, zur Reaktion gebracht wird.

**2.** Verfahren gemäß Anspruch 1, worin die reduktive Aminierung des 2,2,6,6-Tetramethyl-4-piperidons in Abwesenheit eines Lösungsmittels durchgeführt wird.

**3.** Verfahren gemäß Anspruch 1, worin die reduktive Aminierung des 2,2,6,6-Tetramethyl-4-piperidons mit einem wasserfreien Amin der Formel (III) durchgeführt wird.

**4.** Verfahren gemäß Anspruch 1, worin die reduktive Aminierung des 2,2,6,6-Tetramethyl-4-piperidons in Gegenwart einer Wassermenge weniger als 5 Gew.-%, bezogen auf das Reaktionsgemisch, durchgeführt wird.

**5.** Verfahren gemäß Anspruch 1, worin die reduktive Aminierung des 2,2,6,6-Tetramethyl-4-piperidons mit Ethylamin enthaltend 30 % Wasser durchgeführt wird.

**6.** Verfahren gemäß Anspruch 1, worin die reduktive Aminierung des 2,2,6,6-Tetramethyl-4-piperidons mit Hexamethylendiamin enthaltend 15 % Wasser durchgeführt wird.

**7.** Verfahren gemäß Anspruch 1, worin die reduktive Aminierung des 2,2,6,6-Tetramethyl-4-piperidons in Anwesenheit von Platin als Hydrierungskatalysator durchgeführt wird.

**8.** Verfahren gemäß Anspruch 1, worin die reduktive Aminierung des 2,2,6,6-Tetramethyl-4-piperidons bei einer Temperatur von 20 °C bis 120 °C durchgeführt wird.

**9.** Verfahren gemäß Anspruch 1, worin die reduktive Aminierung des 2,2,6,6-Tetramethyl-4-piperidons bei einer Temperatur von 40 °C bis 100 °C durchgeführt wird.

**10.** Verfahren gemaß Anspruch 1, worin die reduktive Aminierung des 2,2,6,6-Tetramethyl-4-piperidons unter einem Wasserstoffdruck von 1 bis 100 bar durchgeführt wird.

**11.** Verfahren gemäß Anspruch 1, worin die reduktive Aminierung des 2,2,6,6-Tetramethyl-4-piperidons unter einem Wasserstoffdruck von 10 bis 80 bar durchgeführt wird.

**12.** Verfahren gemäß Anspruch 1, worin die reduktive Aminierung des 2,2,6,6-Tetramethyl-4-piperidons durchgeführt wird unter Verwendung eines Amins der Formel (III), worin n = 1 oder 2 ist, und falls n = 1 ist, ist R $C_1$-$C_{12}$-Alkyl, $C_5$-$C_9$-Cycloalkyl, Benzyl, Tetrahydrofurfuryl, $C_2$-$C_3$-Alkyl, monosubstituiert durch OH, $C_1$-$C_4$-Alkoxy, $C_1$-$C_4$-Alkylamino, Cyclohexylamino oder Di-($C_1$-$C_4$-alkyl)-amino, oder $C_2$-$C_6$-Alkyl, substituiert durch eine -$NH_2$-Gruppe, oder R ist 2,2,6,6-Tetramethyl-4-piperidyl, und falls n = 2 ist, ist R $C_2$-$C_6$-Alkylen, Cyclohexylen, Methylendicyclohexylen, Cyclohexylendimethylen oder $C_4$-$C_{10}$-Alkylen, das durch 1 oder 2 Sauerstoffatome oder >NH-Gruppen unterbrochen ist.

**13.** Verfahren gemaß Anspruch 1, worin die reduktive Aminierung des 2,2,6,6,-Tetramethyl-4-piperidons durchgeführt wird durch Verwendung eines Amins der Formel (III), worin n = 1 oder 2 ist, und falls n = 1 ist, ist R $C_1$-$C_8$-Alkyl, Cyclohexyl, Tetrahydrofurfuryl, 2,2,6,6-Tetramethyl-4-piperidyl oder $C_2$-$C_3$-Alkyl, das durch $C_1$-$C_4$-Alkyloxy oder durch Di-($C_1$-$C_4$-alkyl)-amino monosubstituiert ist, und falls n = 2 ist, ist R $C_2$-$C_6$-Alkylen oder $C_4$-$C_{10}$-Alkylen, das durch 1 oder 2 Sauerstoffatome oder >NH-Gruppen unterbrochen ist.

**14.** Verfahren gemäß Anspruch 1, worin die reduktive Aminierung des 2,2,6,6-Tetramethyl-4-piperidons durchgeführt wird durch Verwendung eines Amins der Formel (III), worin n = 1 oder 2 ist, und falls n = 1 ist, ist R $C_1$-$C_8$-Alkyl, Cyclohexyl, 2,2,6,6-Tetramethyl-4-piperidyl, 3-Methoxypropyl, 3-Dimethylaminopropyl oder 3-Diethylaminopropyl, und falls n = 2 ist, ist R $C_2$-$C_6$-Alkylen.

**15.** Verfahren gemäß Anspruch 1, worin die reduktive Aminierung des 2,2,6,6-Tetramethyl-4-piperidons durchgeführt wird durch Verwendung eines Amins der Formel (III), worin n = 1 oder 2 ist, und falls n = 1 ist, ist R $C_1$-$C_8$-Alkyl oder 3-Diethylaminopropyl, und falls n = 2 ist, ist R $-(CH_2)_{2-6}-$.

## Revendications

**1.** Procédé pour la préparation de 2,2,6,6-tétraméthyl-4-pipéridylamine de formule (I)

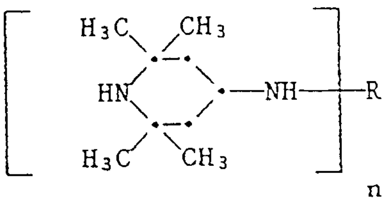

(I)

où n est 1, 2 ou 3 et, si n est 1, R est un groupe alkyle en $C_1$-$C_{18}$, cycloalkyle en $C_5$-$C_{12}$, phénylalkyle en $C_7$-$C_9$, tétrahydrofurfuryle, alkyle en $C_2$-$C_4$ qui est monosubstitué par un groupe OH, alkyloxy en $C_1$-$C_8$, alkylamino en $C_1$-$C_8$, cyclohexylamino ou di(alkyle en $C_1$-$C_8$)amino, ou alkyle en $C_2$-$C_6$ qui est substitué par un groupe $-NH_2$, ou bien R est un groupe 2,2,6,6-tétraméthyl-4-pipéridyle, et, si n est 2, R est un groupe alkylène en $C_2$-$C_{12}$, cyclohexylène, méthylènedicyclohexylène, cyclohexylènediméthylène, xylylène ou alkylène en $C_4$-$C_{12}$ qui est interrompu par 1, 2 ou 3 atomes d'oxygène ou 1 ou 2 groupes >NH ou $>N-CH_3$, et, si n est 3, R est un groupe de formule (II)

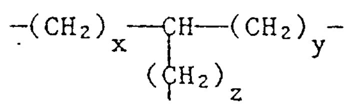

(II)

où x et y sont identiques ou différents et sont des nombres entiers de 2 à 6 et z est zéro ou 1, caractérisé en ce qu'une 2,2,6,6-tétraméthyl-4-pipéridone est amenée à réagir avec une amine de formule (III)

$R-(NH_2)_n$ (III)

où R et n sont tels que définis ci-dessus, en présence d'hydrogène et de platine ou de palladium comme catalyseur d'hydrogénation, non supporté ou supporté sur une matière inerte, en l'absence de solvant ou en présence d'une quantité d'eau inférieure à 5 % en poids par rapport au mélange réactionnel.

**2.** Procédé selon la revendication 1, dans lequel l'amination réductive de la 2,2,6,6-tétraméthyl-4-pipéridone est effectuée en l'absence de solvant.

**3.** Procédé selon la revendication 1, dans lequel l'amination réductive de la 2,2,6,6-tétraméthyl-4-pipéridone est effectuée avec une amine anhydre de formule (III).

**4.** Procédé selon la revendication 1, dans lequel l'amination réductive de la 2,2,6,6-tétraméthyl-4-pipéridone est effectuée en présence d une quantité d'eau inférieure à 5 % en poids par rapport au mélange réactionnel.

**5.** Procédé selon la revendication 1, dans lequel l'amination réductive de la 2,2,6,6-tétraméthyl-4-pipéridone est effectuée avec de l'éthylamine contenant 30 % d'eau.

**6.** Procédé selon la revendication 1, dans lequel l'amination réductive de la 2,2,6,6-tétraméthyl-4-pipéridone est effectuée avec de l'hexaméthylènediamine contenant 15 % d'eau.

7. Procédé selon la revendication 1, dans lequel l'amination réductive de la 2,2,6,6-tétraméthyl-4-pipéridone est effectuée en présence de platine comme catalyseur d'hydrogénation.

8. Procédé selon la revendication 1, dans lequel l'amination réductive de la 2,2,6,6-tétraméthyl-4-pipéridone est effectuée à une température de 20° à 120°C.

9. Procédé selon la revendication 1, dans lequel l'amination réductive de la 2,2,6,6-tétraméthyl-4-pipéridone est effectuée à une température de 40° à 100°C.

10. Procédé selon la revendication 1, dans lequel l'amination réductive de la 2,2,6,6-tétraméthyl-4-pipéridone est effectuée sous une pression d'hydrogène de 1 à 100 bars.

11. Procédé selon la revendication 1, dans lequel l'amination réductive de la 2,2,6,6-tétraméthyl-4-pipéridone est effectuée sous une pression d'hydrogène de 10 à 80 bars.

12. Procédé selon la revendication 1, dans lequel l'amination réductive de la 2,2,6,6-tétraméthyl-4-pipéridone est effectuée en utilisant une amine de formule (III) dans laquelle n est 1 ou 2 et, si n est 1, R est un groupe alkyle en $C_1$-$C_{12}$, cycloalkyle en $C_5$-$C_9$, benzyle, tétrahydrofurfuryle, alkyle en $C_2$-$C_3$ qui est monosubstitué par un groupe OH, alkyloxy en $C_1$-$C_4$, alkylamino en $C_1$-$C_4$, cyclohexylamino ou di(alkyle en $C_1$-$C_4$)amino, ou alkyle en $C_2$-$C_6$ qui est substitué par un groupe $-NH_2$, ou bien R est un groupe 2,2,6,6-tétraméthyl-4-pipéridyle, et, si n est 2, R est un groupe alkylène en $C_2$-$C_6$, cyclohexylène, méthylènedicyclohexylène, cyclohexylènediméthylène ou alkylène en $C_4$-$C_{10}$ qui est interrompu par 1 ou 2 atomes d'oxygène ou groupes >NH.

13. Procédé selon la revendication 1, dans lequel l'amination réductive de la 2,2,6,6-tétraméthyl-4-pipéridone est effectuée en utilisant une amine de formule (III) dans laquelle n est 1 ou 2 et, si n est 1, R est un groupe alkyle en $C_1$-$C_8$, cyclohexyle, 2,2,6,6-tétraméthyl-4-pipéridyle ou alkyle en $C_2$-$C_3$ qui est monosubstitué par un groupe alkyloxy en $C_1$-$C_4$ ou di(alkyle en $C_1$-$C_4$)amino, et, si n est 2, R est un groupe alkylène en $C_2$-$C_6$ ou alkylène en $C_4$-$C_{10}$ qui est interrompu par 1 ou 2 atomes d'oxygène ou groupes >NH.

14. Procédé selon la revendication 1, dans lequel l'amination réductive de la 2,2,6,6-tétraméthyl-4-pipéridone est effectuée en utilisant une amine de formule (III) dans laquelle n est 1 ou 2 et, si n est 1, R est un groupe alkyle en $C_1$-$C_8$, cyclohexyle, 2,2,6,6-tétraméthyl-4-pipéridyle, 3-méthoxypropyle, 3-diméthylaminopropyle ou 3-diéthylaminopropyle, et, si n est 2, R est un groupe alkylène en $C_2$-$C_6$.

15. Procédé selon la revendication 1, dans lequel l'amination réductive de la 2,2,6,6-tétraméthyl-4-pipéridone est effectuée en utilisant une amine de formule (III) dans laquelle n est 1 ou 2 et, si n est 1, R est un groupe alkyle en $C_1$-$C_8$ ou 3-diéthylaminopropyle et, si n est 2, R est $-(CH_2)_{2-6}-$.